(19) Europäisches Patentamt
European Patent Office
Office européen des brevets

(11) Publication number: **0 058 071**
**B1**

# (12) EUROPEAN PATENT SPECIFICATION

(45) Date of publication of patent specification: **23.07.86**

(51) Int. Cl.$^4$: **C 07 D 213/65** // C07D213/89

(21) Application number: **82300604.4**

(22) Date of filing: **08.02.82**

## (54) Process and intermediates for preparing pirbuterol.

(30) Priority: **09.02.81 US 232923**

(43) Date of publication of application:
**18.08.82 Bulletin 82/33**

(45) Publication of the grant of the patent:
**23.07.86 Bulletin 86/30**

(84) Designated Contracting States:
**AT BE CH DE FR GB IT LI LU NL SE**

(56) References cited:
**US-A-4 031 108**

**KATRITZKY, Chemistry of the HETEROCYCLIC N-OXIDES, 1971, pp. 353-361 (A.P.Ed.)**
**JACS, 76 (1954), pp. 1286-1291**

(73) Proprietor: **PFIZER INC.**
**235 East 42nd Street**
**New York, N.Y. 10017 (US)**

(72) Inventor: **Cue, Berkeley Wendell, Jr.**
**266 Stoddards Wharf Drive**
**Gales Ferry Connecticut (US)**
Inventor: **Massett, Stephen Sargent**
**78 Brandegee Avenue**
**Groton Connecticut (US)**

(74) Representative: **Wood, David John et al**
**Pfizer Limited Ramsgate Road**
**Sandwich Kent CT13 9NJ (GB)**

**EP 0 058 071 B1**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European patent convention).

Courier Press, Leamington Spa, England.

**Description**

This invention relates to a process for the preparation of pirbuterol, pirbuterol having the formula

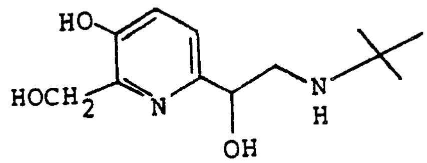

and certain intermediates therefor.

Pirbuterol and its bronchodilator activity were originally described by Barth in a series of U.S. Patents (3,700,681; 3,763,173; 3,772,314; 3,786,160). At that time, pirbuterol was synthesized as follows:

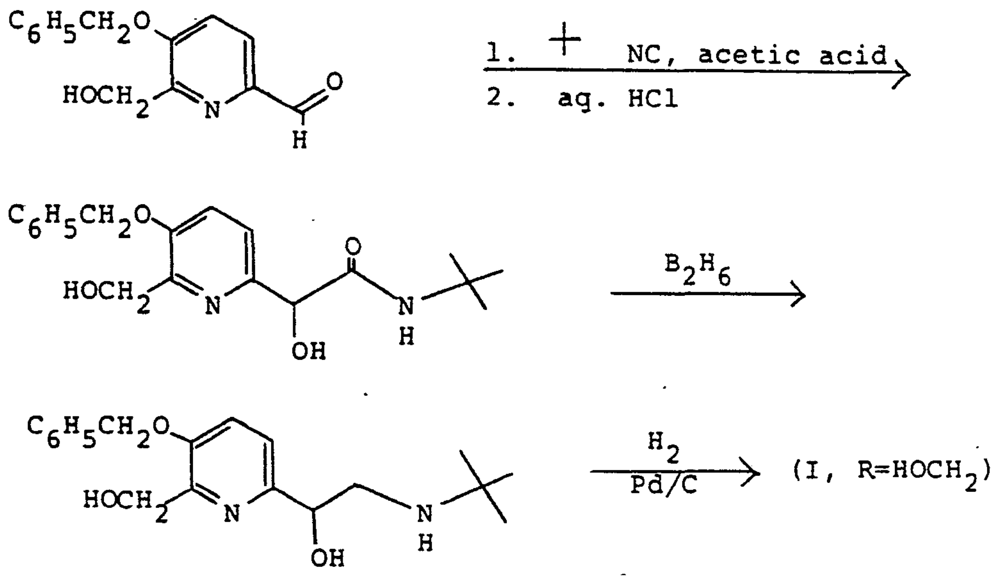


The pirbuterol analogs having hydrogen, methyl, or methanesulfonylmethyl, in place of hydroxymethyl, and their bronchodilator activity, were disclosed by Jen and Kaiser in U.S. Patent 3,952,101. These compounds were prepared from the corresponding aldehydes by a reaction sequence fully analogous with that of the earlier pirbuterol process.

Subsequently, an alternative, preferred process for the synthesis of pirbuterol was described by Nakanishi (U.S. Patents 3,948,919; 4,031,108), exemplified as follows:

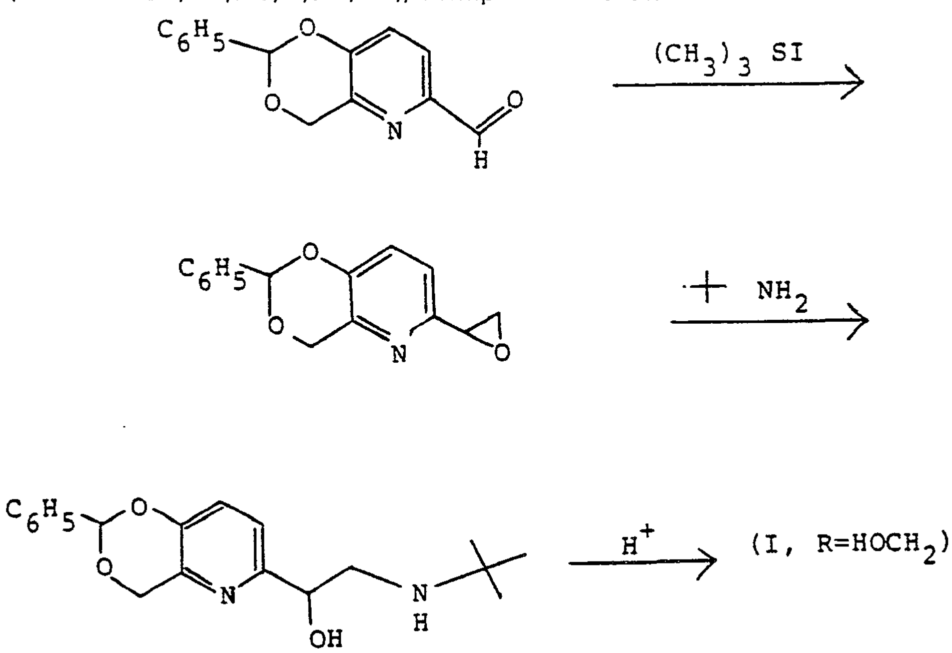

Improvements and alternatives to this process have also been described, *viz.,* isolation of the pirbuterol precursor as a maleate salt (Carroll *et al.,* U.S. Patent 4,011,231) and hydrogenolysis rather than hydrolysis as the final stage (Argentina Patent 214005, Luxembourg Patent 79564).

The latter process still suffers from the disadvantage of generating noxious sulfide by-products in the preparation of epoxide. Even under the best of conditions, traces of sulfur can carry through and increase the catalyst level when protecting groups are removed by hydrogenolysis, the preferred route when the free base form of pirbuterol is desired. Furthermore, the epoxide is relatively unreactive towards the *tert*-butylamine reagent, even under pressure at elevated temperatures requiring large excess of the reagent and a relatively long time to achieve complete reaction.

A further disadvantage of the improved process is the polar nature of intermediate amino alcohol, making this intermediate difficult to isolate in pure form.

Recently pirbuterol has been discovered to also have utility for the treatment of congestive heart failure (Taylor, U.S. Patent 4,175,128).

In a chemical transformation related to one of the process steps of the present invention, Benderly *et al.,* [Can. J. Chem. 56, pp.2673—2676 (1978)] have reported the conversion of 2-vinylpyridine to 2-(1,2-epoxyethyl)-pyridine N-oxide by the action of *m*-chloroperbenzoic acid on 2-vinylpyridine.

The present invention provides a process for preparing pirbuterol, or a pharmaceutically acceptable acid addition salt thereof, which comprises the solvolysis of a compound of the formula:—

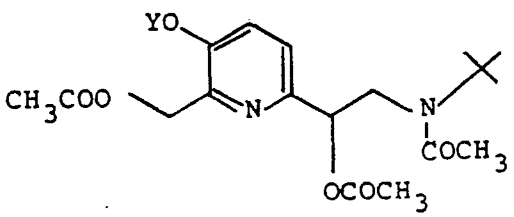

(VIII)

wherein Y is benzyl or $CH_3CO$—;
or, alternatively, when Y is benzyl, a combination of solvolysis and hydrogenolysis of the compound (VIII); followed by, optionally, conversion of the pirbuterol into a pharmaceutically acceptable acid addition salt by reaction with a suitable acid.

The invention also provides a process for the preparation of pirbuterol which comprises

(a) heating a compound of the formula

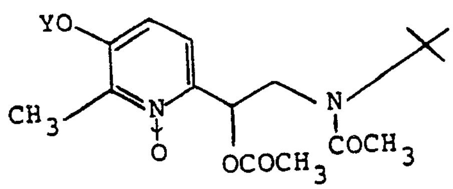

(VI)

in which Y is benzyl or $CH_3CO$—,
in acetic anhydride to form a compound of the formula

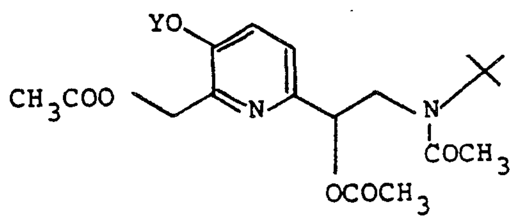

(VIII)

wherein Y is as defined above, followed by

(b) solvolysis or when Y is benzyl a combination of solvolysis and hydrogenolysis of the compound (VIII) to form pirbuterol, followed by, optionally, conversion of the pirbuterol into a pharmaceutically acceptable acid addition salt by reaction with a suitable acid.

These processes and the starting materials can be illustrated by the following chemical steps:

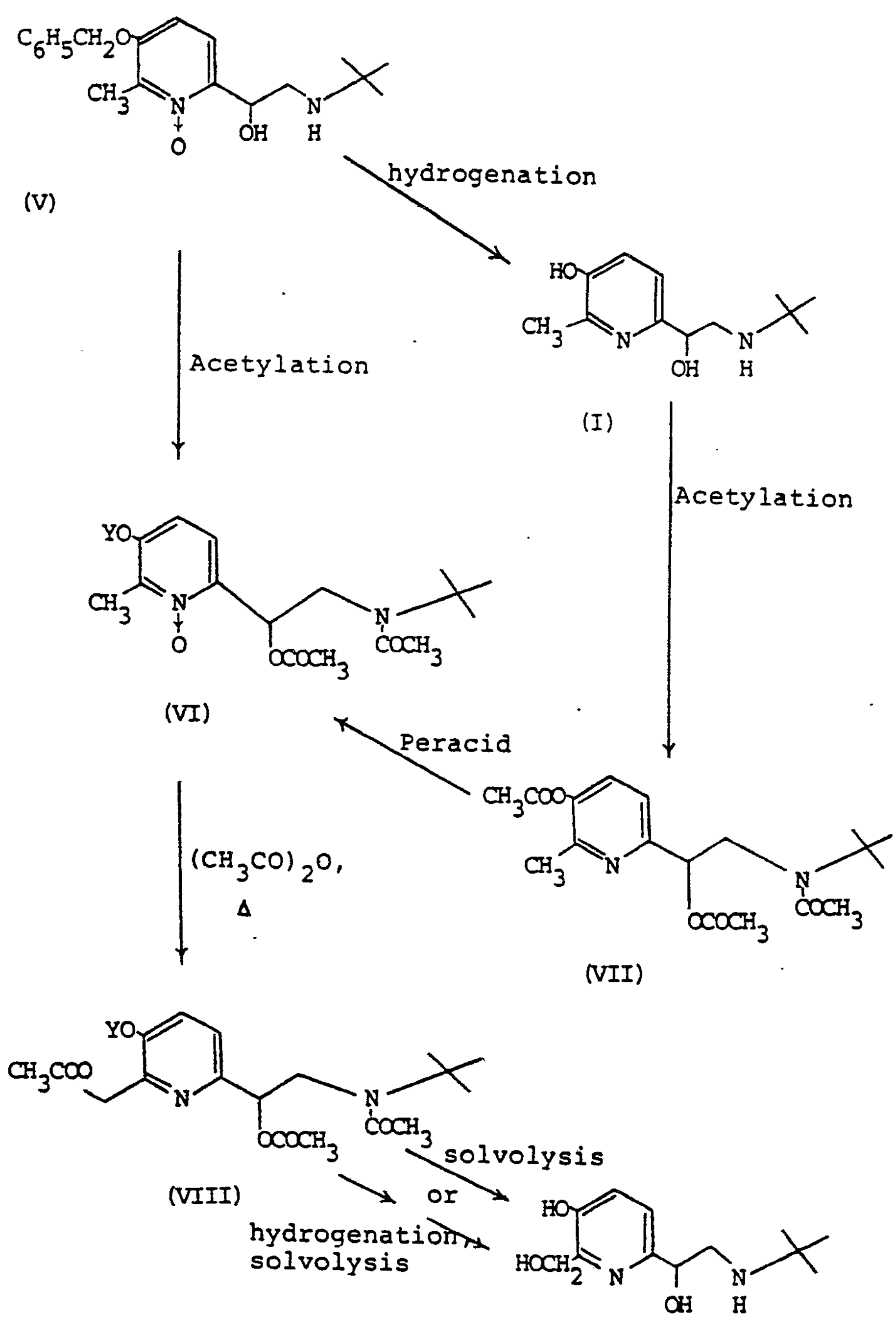


(V)

(I)

(VI)

(VII)

(VIII)

(I) Pirbuterol

wherein in the formulae (VI) and (VIII), Y is benzyl or acetyl depending on whether precursor (V) or (VII) is used. The intermediate (V) is easily produced from the readily available corresponding pyridine carbaldehyde.

Conversion of the amine (V) to compound (I) can be accomplished by hydrogenolysis over a noble metal catalyst in a reaction-inert solvent. The temperature of the hydrogenolysis is not critical, e.g. 0—100°C. can be used, but is conveniently carried out at ambient temperature avoiding the costs of cooling or heating. The pressure of the hydrogenation is also not critical (e.g. pressures of subatmospheric to 1378951 Pa or more can be used), but relatively low pressures (e.g. 137895—482633 Pa) are generally used, since the reaction proceeds at a reasonable rate without requiring the more elaborate equipment characteristic of high pressure hydrogenations. The noble metal catalysts employed in the present invention include platinum, palladium, ruthenium and rhodium, either of the supported or non-supported

type, as well as the known catalytic compounds thereof such as oxides, chlorides. Examples of suitable catalyst supports include carbon and barium sulfate. In the present instances a preferred catalyst is palladium on carbon (1 to 10% of Pd by weight). The hydrogenation solvent is also not critical. It should not react with reactants or product (i.e. it should be reaction inert). Suitable solvents include ($C_1$—$C_4$) alcohols, particularly methanol. Other solvents such as tetrahydrofuran or dioxane can, of course, be used either alone or in combination with each other and/or the above mentioned alcohols. Higher boiling solvents, (e.g. ethylene glycol, diglyme) can also be used, but are not favored because more energy is required to remove them from the reaction mixture. In order to minimize or avoid hydrogenolysis of the 1-hydroxy-2-tert-butylaminoethyl side chain to a 2-tert-butylethyl group, it is preferred that the hydrogenolysis be carried out in the presence of a small amount of water (e.g. from 1 to 30 molar equivalents).

The acetylations to produce compounds of the formulae (VI) and (VII) are typically carried out under mild conditions with at least 2 to 3 molar equivalents, respectively, of an acetylating agent (e.g. acetyl chloride, acetic anhydride), in the presence of a *tert*-amine such as triethylamine, in an amount usually at least molarly equivalent to the acetylating agent. The reaction is carried out in an inert solvent, such as methylene chloride, ethyl acetate or the like. The temperature is non-critical in the case of the acetylation of (I) and can be in the range of 0—100°C. When ethyl acetate is used as solvent, it is convenient to employ the reflux temperature of the reaction mixture. However, in the case of the acetylation of (V), lower temperature, e.g. 0—30°C., are preferred in order to avoid or minimize side reactions involving the N-oxide group.

The oxidation of the pyridine compound (VII) to the pyridine-N-oxide (VI) is carried out by the action of a peracid, conventionally *m*-chloroperbenzoic acid, by conventional techniques.

The key reaction in the sequence, rearrangement of the pyridine-N-oxide (VI) to the acetoxymethylpyridine (VIII) is typically carried out by heating in excess acetic anhydride. The temperature employed is generally in the range 120—160°C., conveniently at the reflux temperature of the reaction mixture (about 140°C.), but preferably under elevated pressure when the reaction is carried out at or above the atmospheric boiling point of the reaction mixture, thus avoiding the unnecessary cost of condensing and reheating acetic anhydride.

The final solvolysis, or hydrogenolysis-solvolysis, is carried out as follows:

The solvolysis is carried out in aqueous or alcoholic media, with or without cosolvents, usually in the presence of an acid catalyst. Particularly suitable is a combination of methanol and hydrogen chloride from which the intermediate is readily isolated in the form of the hydrochloride salt. Temperature is not critical; e.g. temperatures of 0—50°C. or higher can be ued, but the reaction is conveniently carried out at ambient temperature. Acid catalyzed solvolysis is preferred over base catalyzed solvolysis, since the products have some tendency to degrade under basic conditions. When solvolysis-hydrogenolysis is used, the final stage hydrolenolysis can be carried out according to the method detailed above. Over hydrogenation which would convert the 6-hydroxymethyl group to methyl should be avoided

The present invention is illustrated by the following Example:

## Example

A. 3-Acetoxy-2-methyl-6-[1-acetoxy-2-(N-*tert*-butylacetamido)ethyl]pyridine (VII)

To a suspension of 2-(1-hydroxy-2-*tert*-butylaminoethyl)-5-hydroxy-6-methylpyridine dihydrochloride (7.70 g., 0.026 mole) in 300 ml. of ethyl acetate and 7.7 ml. of triethylamine, 10.8 ml. of acetic anhydride was added and the solution was heated at the reflux temperature for 30 hours. An additional 5 ml. of acetic anhydride was added the reaction mixture and heating was continued for 24 hours, then allowed to cool to room temperature. The reaction mixture was poured into aqueous sodium bicarbonate solution (100 g. $NaHCO_3$ in 700 ml. $H_20$) and the resulting mixture was stirred for 1 hour. The ethyl acetate layer was separated, washed with aqueous sodium bicarbonate, then water and dried over magnesium sulfate. Evaporation of the dried ethyl acetate solution *in vacuo* gave 3-acetoxy-2-methyl-6-[1-acetoxy-2-(N-*tert*-butylacetamido)ethyl]pyridine, the product 6.0 g. (66%); m.p. 124.5—127.5°C.

| | | | |
|---|---|---|---|
| Anal. Calcd. for $C_{18}H_{26}N_2O_5$: | C, 61.70; | H, 7.48; | N, 8.00 |
| Found: | C, 61.93; | H, 7.39; | N, 8.44 |

B. 3-Acetoxy-2-methyl-6-[1-acetoxy-2-(N-*tert*-butylacetamido)ethyl]pyridine N-Oxide (VI, Y=$CH_3CO$)

A solution of 3-acetoxy-2-methyl-6-[1-acetoxy-2-(N-*tert*-butylacetamido)ethyl]pyridine (5.50 g., 0.0157 mole) and *m*-chloroperbenzoic acid (85% technical grade, 3.90 g., 0.0192 mole) in 150 ml. of chloroform was stirred at room temperature for 24 hours. After washing the reaction mixture with two portions of aqueous sodium bicarbonate solution, the dried ($MgSO_4$) chloroform solution was evaporated to give 2.9 g. (50%) of 3-acetoxy-2-methyl-6-[1-acetoxy-2-(N-*tert*-butylacetamido)ethyl]pyridine N-oxide; m.p. 113—116.5°C. after recrystallization from ether.

| | | | |
|---|---|---|---|
| Anal. Calcd. for $C_{18}H_{26}N_2O_6$: | C, 59.00; | H, 7.15; | N, 7.65 |
| Found: | C, 59.25; | H, 7.08; | N, 8.14 |

C. 3-Acetoxy-2-acetoxymethyl-6-[1-acetoxy-2-(N-*tert*-butylacetamido)ethyl]pyridine (VIII)

A solution of 1.0 g. (0.0027 mole) of 3-acetoxy-2-methyl-6-[1-acetoxy-2-(N-*tert*-butylacetamido)ethyl]pyridine N-oxide in 10 ml. of acetic anhydride was heated at the reflux temperature for 2.5 hours, then was allowed to cool to room temperature and stirred at this temperature overnight. The reaction mixture was poured into a two phase mixture of ethyl acetate and aqueous sodium bicarbonate and stirred for 1 hour. The organic layer was separated, dried over anhydrous megnesium sulfate, filtered and evaporated to dryness *in vacuo* to give a brown oil which was purified by column chromatography on silica gel with ether as the eluant to give 300 mg. (27%) of the desired product; m.p. 60—64°C.

| | | | |
|---|---|---|---|
| Anal. Calcd. for $C_{20}H_{28}N_2O_7$: | C, 58.81; | H, 6.91; | N, 6.86 |
| Found: | C, 59.02; | H, 6.83; | N, 7.30 |

D. Pirbuterol Hydrochloride

A solution of 2.0 g., (0.005 mole) of 2-acetoxymethyl-3-acetoxy-6-(1-acetoxy-2-N-acetyl-*tert*-butylaminoethyl)pyridine in 50 ml. of methanol containing 1 ml. of concentrated hydrochloric acid was heated to reflux for 12 hours. The solvents were removed *in vacuo* and the residue recrystallized from ethyl acetate to give pirbuterol hydrochloride (900 mg., 58%) m.p. 172—175°C. (dec.), NMR and tlc in 6:3:1 EtOAz: $CH_3OH$: $Et_2NH$ identical to pirbuterol. Prolonged heating results in the formation of 2-hydroxymethyl-3-hydroxy-6-(1-hydroxy-2-aminoethyl)pyridine, m.p. 184—186° dec.

**Claims for the Contracting States: BE CH DE FR GB IT LI LU NL SE**

1. A process for preparing pirbuterol, or a pharmaceutically acceptable acid addition salt thereof, which comprises the solvolysis of a compound of the formula:—

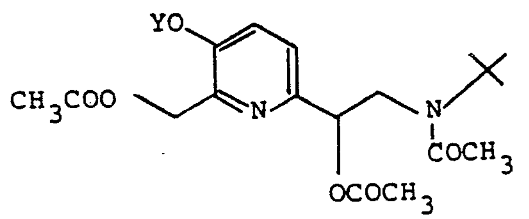

(VIII)

wherein Y is benzyl or $CH_3CO$—;
or, alternatively, when Y is benzyl, a combination of solvolysis and hydrogenolysis of the compound (VIII); followed by, optionally, conversion of the pirbuterol into a pharmaceutacally acceptable acid addition salt by reaction with a suitable acid.

2. A process for the preparation of pirbuterol which comprises

(a) heating a compound of the formula

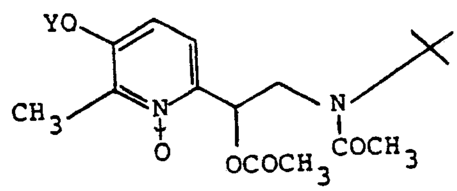

(VI)

in which Y is benzyl or $CH_3CO$—,
in acetic anhydride to form a compound of the formula

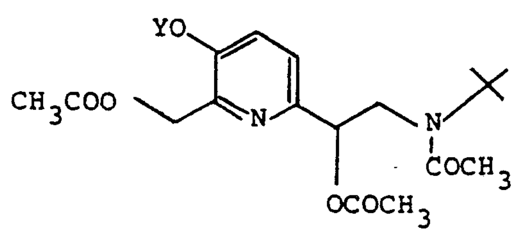

(VIII)

wherein Y is as defined above, followed by

(b) solvolysis or when Y is benzyl a combination of solvolysis and hydrogenolysis of the compound (VIII) to form pirbuterol, followed by, optionally, conversion of the pirbuterol into a pharmaceutically acceptable acid addition salt by reaction with a suitable acid.

3. A process according to claim 2, wherein step (a) is carried out at a temperature of from 120—160°C.

4. The process of claim 1, 2 or 3 wherein Y is $CH_3CO$— and solvolysis alone with a combination of methanol and hydrochloric acid is used.

5. The process of claim 1, 2 or 3 wherein Y is benzyl and a combination of hydrogenolysis and solvolysis is used.

6. A compound of the formula

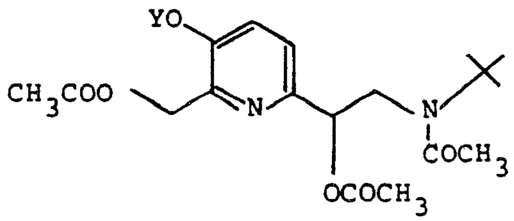

(VIII)

wherein Y is benzyl or —$COCH_3$.

7. The compound of claim 6 wherein Y is benzyl.

8. The compound of claim 6 wherein Y is acetyl.

**Claims for the Contracting State: AT**

1. A process for preparing pirbuterol, or a pharmaceutically acceptable salt thereof, which comprises the solvolysis of a compound of the formula:—

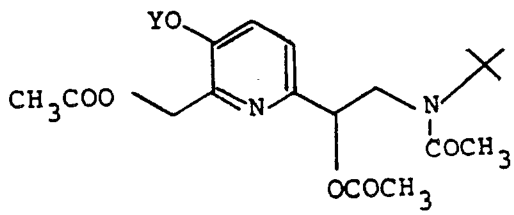

(VIII)

wherein Y is benzyl or $CH_3CO$—;
or, alternatively, when Y is benzyl, a combination of solvolysis and hydrogenolysis of the compound (VIII); followed by, optionally, conversion of the pirbuterol into a pharmaceutacally acceptable acid addition salt by reaction with a suitable acid.

2. A process for the preparation of pirbuterol which comprises

(a) heating a compound of the formula

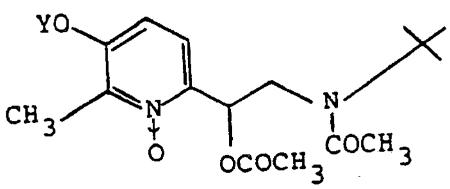

(VI)

in which Y is benzyl or $CH_3CO$,
in acetic anhydride to form a compound of the formula

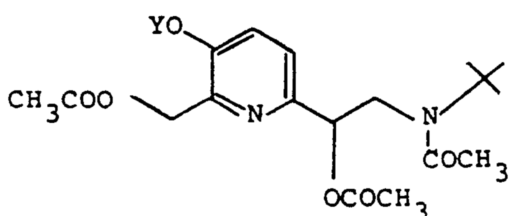

(VIII)

wherein Y is as defined above, followed by

(b) solvolysis or when Y is benzyl a combination of solvolysis and hydrogenolysis of the compound (VIII) to form pirbuterol, followed by, optionally, conversion of the pirbuterol into a pharmaceutically acceptable acid addition salt by reaction with a suitable acid.

3. A process according to claim 2, wherein step (a) is carried out at a temperature of from 120—160°C.

4. The process of claim 1, 2 or 3 wherein Y is $CH_3CO$ and solvolysis alone with a combination of methanol and hydrochloric acid is used.

5. The process of claim 1, 2 or 3 wherein Y is benzyl and a combination of hydrogenolysis and solvolysis is used.

**Patentansprüche für die Vertragsstaaten: BE CH DE FR GB IT LI LU NL SE**

1. Verfahren zur Herstellung von Pirbuterol oder eines pharmazeutisch annehmbaren Salzes hievon, welches die Solvolyse einer Verbindung der Formel

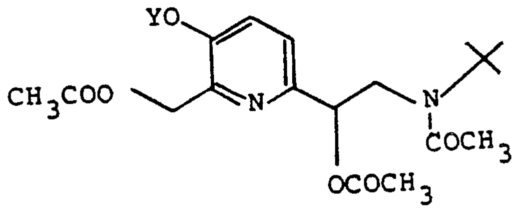

(VIII.)

worin Y Benzyl oder $CH_3CO$— ist, oder andererseits, wenn Y Benzyl ist, eine Kombination von Solvolyse und Hydrogenolyse der Verbindung (VIII), gegebenenfalls gefolgt von der Überführung von Pirbuterol in ein pharmazeutisch annehmbares Säureadditionssalz durch Umsetzen mit einer geeigneten Säure umfaßt.

2. Verfahren zur Herstellung von Pirbuterol, welches

(a) das Erhitzen einer Verbindung der Formel

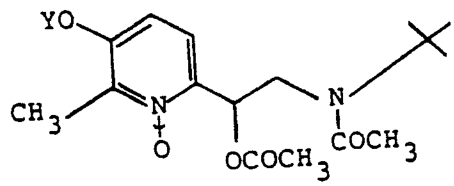

(VI)

worin Y Benzyl oder $CH_3CO$— ist, in Essigsäureanhydrid unter Bildung einer Verbindung der Formel

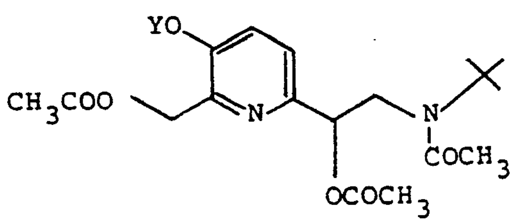

(VIII)

worin Y wie oben definiert ist, gefolgt von

(b) Solvolyse oder, wenn Y Benzyl ist, einer Kombination von Solvolyse und Hydrogenolyse der Verbindung (VIII) unter Bildung von Pirbuterol, gegebenenfalls gefolgt von Überführung des Pirbuterols in ein pharmazeutisch annehmbares Säureadditionssalz durch Reaktion mit einer geeigneten Säure umfaßt.

3. Verfahren nach Anspruch 2, worin Schritt (a) bei einer Temperatur von 120 bis 160°C durchgeführt wird.

4. Verfahren nach Anspruch 1, 2 oder 3, worin Y $CH_3CO$— ist und Solvolyse allein mit einer Kombination von Methanol und Salzsäure angewandt wird.

5. Verfahren nach Anspruch 1, 2 oder 3, worin Y Benzyl ist und eine Kombination von Hydrogenolyse und Solvolyse angewandt wird.

6. Verbindung der Formel

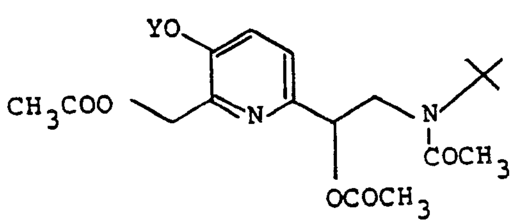

(VIII)

worin Y Benzyl oder —$COCH_3$ ist.

7. Verbindung nach Anspruch 6, worin Y Benzyl ist.

8. Verbindung nach Anspruch 6, worin Y Acetyl ist.

**Patentansprüche für den Vertragsstaat: AT**

1. Verfahren zur Herstellung von Pirbuterol oder eines pharmazeutisch annehmbaren Salzes hievon, welches die Solvolyse einer Verbindung der Formel

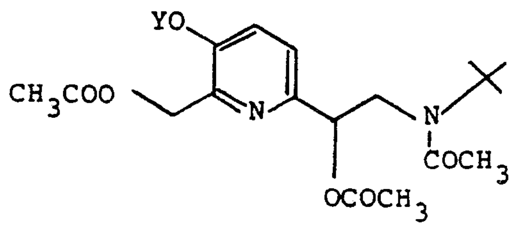

(VIII)

worin Y Benzyl oder $CH_3CO$— ist, oder andererseits, wenn Y Benzyl ist, eine Kombination von Solvolyse und Hydrogenolyse der Verbindung (VIII), gegebenenfalls gefolgt von der Überführung von Pirbuterol in ein pharmazeutisch annehmbares Säureadditionssalz durch Umsetzen mit einer geeigneten Säure umfaßt.

2. Verfahren zur Herstellung von Pirbuterol, welches

(a) das Erhitzen einer Verbindung der Formel

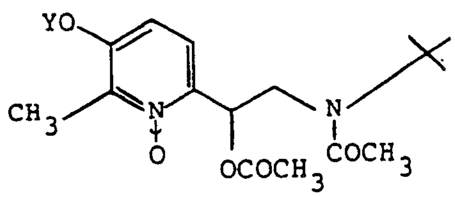

(VI)

worin Y Benzyl oder $CH_3CO$— ist, in Essigsäureanhydrid unter Bildung einer Verbindung der Formel

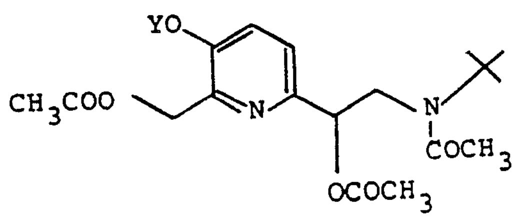

(VIII)

worin Y wie oben definiert ist, gefolgt von

(b) Solvolyse oder, wenn Y Benzyl ist, einer Kombination von Solvolyse und Hydrogenolyse der Verbindung (VIII) unter Bildung von Pirbuterol, gegebenenfalls gefolgt von Überführung des Pirbuterols in ein pharmazeutisch annehmbares Säureadditionssalz durch Reaktion mit einer geeigneten Säure umfaßt.

3. Verfahren nach Anspruch 2, worin Schritt (a) bei einer Temperatur von 120 bis 160°C durchgeführt wird.

4. Verfahren nach Anspruch 1, 2 oder 3, worin Y $CH_3CO$— ist und Solvolyse allein mit einer Kombination von Methanol und Salzsäure angewandt wird.

5. Verfahren nach Anspruch 1, 2 oder 3, worin Y Benzyl ist und eine Kombination von Hydrogenolyse und Solvolyse angewandt wird.

**Revendications pour les Etats contractants: BE CH DE FR GB IT LI LU NL SE**

1. Procédé de préparation de pributérol ou d'un sel d'addition d'acide pharmaceutiquement acceptable de ce composé, qui comprend la solvolyse d'un composé de formule:

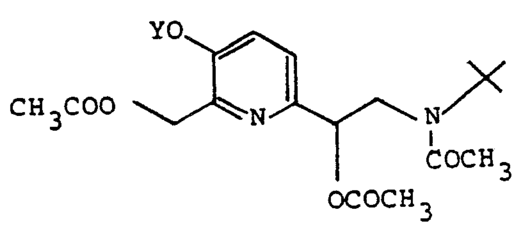

(VIII)

dans laquelle Y est le groupe benzyle ou $CH_3CO$—;
ou bien, à titre de variante, lorsque Y est le groupe benzyle, l'action combinée de la solvolyse et de

l'hydrogénolyse du composé (VIII); suivie, à titre facultatif, de la transformation du pirbutérol en un sel d'addition d'acide pharmaceutiquement acceptable par réaction avec un acide approprié.

2. Procédé de préparation du pirbutérol, qui consiste

(a) à chauffer un composé de formule

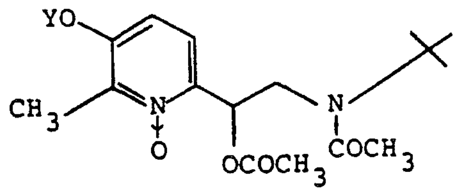

(VI)

dans laquelle Y est le groupe benzyle ou $CH_3CO$—, dans l'anhydride acétique pour former un composé de formule

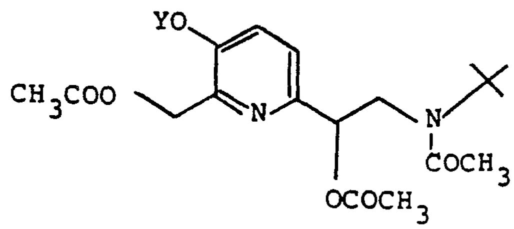

(VIII)

dans laquelle Y a la définition donnée ci-dessus, l'opération étant suivie

(b) d'une solvolyse ou bien, lorsque Y est un groupe benzyle, d'une action combinée de solvolyse et d'hydrogénolyse du composé (VIII) pour former le pirbutérol, suivie, le cas échéant, d'une transformation du pirbutérol en un sel d'addition d'acide pharmaceutiquement acceptable par réaction avec un acide convenable.

3. Procédé suivant la revendication 2, dans lequel l'etape (a) est conduite à une température de 120 à 160°C.

4. Procédé suivant la revendication 1, 2 ou 3, dans lequel Y est un groupe $CH_3CO$— et on utilise la solvolyse seule avec une association entre méthanol et acide chlorhydrique.

5. Procédé suivant la revendication 1, 2 ou 3, dans lequel Y est le groupe benzyle et on utilise une action combinée d'hydrogénolyse et de solvolyse.

6. Composé de formule

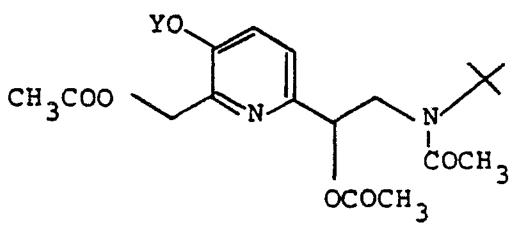

(VIII)

dans laquelle Y est le groupe benzyle ou —$COCH_3$.

7. Composé suivant la revendication 6, dans lequel Y est le groupe benzyle.

8. Composé suivant la revendication 6, dans lequel Y est le groupe acétyle.

**Revendications pour l'Etat contactant: AT**

1. Procédé de préparation du pirbutérol ou d'un sel pharmaceutiquement acceptable de ce composé, qui comprend la solvolyse d'un composé de formule:

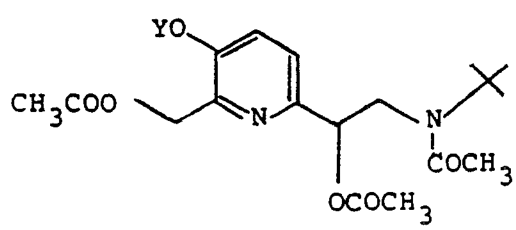

(VIII)

dans laquelle Y est le groupe benzyle ou $CH_3CO$—;

ou bien, à titre de variante, lorsque Y est le groupe benzyle, l'action combinée de la solvolyse et de

l'hydrogénolyse du composé (VIII); suivie, à titre facultatif, de la transformation du pirbutérol en un sel d'addition d'acide pharmaceutiquement acceptable par réaction avec un acide approprié.

2. Procédé de préparation du pirbutérol, qui consiste

(a) à chauffer un composé de formule

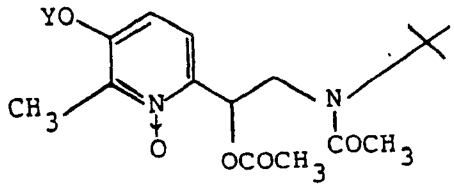

(VI)

dans laquelle Y est le groupe benzyle ou $CH_3CO$—, dans l'anhydride acétique pour former un composé de formule

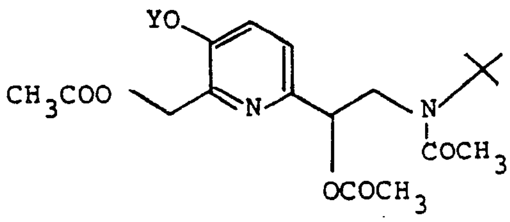

(VIII)

dans laquelle Y a la définition donnée ci-dessus, l'opération étant suivie

(b) d'une solvolyse ou bien, lorsque Y est un groupe benzyle, d'une action combinée de solvolyse et d'hydrogénolyse du composé (VIII) pour former le pirbutérol, suivie, le cas échéant, d'une transformation du pirbutérol en un sel d'addition d'acide pharmaceutiquement acceptable par réaction avec un acide convenable.

3. Procédé suivant la revendication 2, dans lequel l'etape (a) est mise en oeuvre à une température de 120 à 160°C.

4. Procédé suivant la revendication 1, 2 ou 3, dans lequel Y est un groupe $CH_3CO$ et la solvolyse seule est utilisée avec une association entre méthanol et acide chlorhydrique.

5. Procédé suivant la revendication 1, 2 ou 3, dans lequel Y est le groupe benzyle et on utilise une action combinée d'hydrogénolyse et de solvolyse.